(19) Europäisches Patentamt
European Patent Office
Office européen des brevets

(11) **EP 1 666 541 B1**

(12) **EUROPEAN PATENT SPECIFICATION**

(45) Date of publication and mention
of the grant of the patent:
**21.12.2011 Bulletin 2011/51**

(51) Int Cl.:
*C09C 1/28* (2006.01)     *C09C 3/06* (2006.01)
*C09D 11/00* (2006.01)    *C09D 201/00* (2006.01)
*C08L 101/00* (2006.01)   *C08K 9/02* (2006.01)

(21) Application number: **04787954.9**

(22) Date of filing: **21.09.2004**

(86) International application number:
**PCT/JP2004/013771**

(87) International publication number:
**WO 2005/028566 (31.03.2005 Gazette 2005/13)**

(54) **BLACK BRIGHT PIGMENT, AND COSMETIC, COATING COMPOSITION, RESIN COMPOSITION AND INK COMPOSITION COMPRISING THE SAME**

SCHWARZAUFHELLPIGMENT UND ES ENTHALTENDE KOSMETISCHE, BESCHICHTUNGS-, HARZ- UND TINTENZUSAMMENSETZUNG

PIGMENT NOIR BRILLANT, PRODUIT COSMETIQUE, COMPOSITION DE REVETEMENT, COMPOSITION DE RESINE ET COMPOSITION D'ENCRE CONTENANT LEDIT PIGMENT

(84) Designated Contracting States:
**DE FR GB IT**

(30) Priority: **22.09.2003 JP 2003329239**

(43) Date of publication of application:
**07.06.2006 Bulletin 2006/23**

(73) Proprietor: **Nippon Sheet Glass Co.,Ltd.
Tokyo 105-8552 (JP)**

(72) Inventors:
• **YANAGASE, Shigeru,
c/o NIPPON SHEET GLASS CO., LTD
Tokyo 105-8552 (JP)**
• **YOKOI, Koji,
c/o NIPPON SHEET GLASS COMPANY, LTD
Tokyo 105-8552 (JP)**
• **INO, Juichi,
c/o NIPPON SHEET GLASS COMPANY, LTD
Tokyo 105-8552 (JP)**
• **NIIDA, Haruki,
c/o NIPPON SHEET GLASS COMPANY, LTD
Tokyo 105-8552 (JP)**

(74) Representative: **Teipel, Stephan et al
Lederer & Keller
Patentanwälte
Unsöldstrasse 2
80538 München (DE)**

(56) References cited:
**EP-A- 0 763 573      WO-A-00/17277
WO-A-03/006558      JP-A- 9 077 512
JP-A- 2001 207 077   JP-A- 2002 526 591
JP-A- 2003 525 315**

EP 1 666 541 B1

## Description

### Technical Field

The present invention relates to a flaky (flake particle-like) black bright pigment to be added to a cosmetic, a coating material, a coating film, a resin molding composition, a resin molded product, or an ink.

### Background Art

Conventionally, as a flaky black color pigment having a brightness, there have been known mica coated with carbon black (Patent Document No. 1: Japanese Examined Patent Publication No. 39-13216), mica coated with iron oxyhydroxide or magnetite (triiron tetroxide=iron oxide black)(Patent Document No. 2: Japanese Patent Application Laid-Open No. 49-128027), a method of reducing mica coated with titanium dioxide or a compounded oxide of a metal oxide and titanium dioxide into titaniummonoxide (Patent Document No. 3: Japanese Patent Application Laid-Open No. 58-164653), a method of coating titanium dioxide-coated mica with triiron tetroxide (Patent Document No. 4: Japanese Patent Application Laid-Open No. 61-19666), and mica coated with black iron oxide defined by the general formula: $(Fe_2O_3)_x(FeO)_y$ wherein $x : y = (1.5 \text{ to } 5) : 1$ (Patent Document No. 5: Japanese Patent Application Laid-Open No. 4-145168). Also, Patent Document No. 6: Japanese Patent Application Laid-Open No. 2002-30232 describes a metallic pigment comprising glass powder coated with a nickel alloy with a thickness of 0.04 $\mu$m or thinner and showing black appearance.

WO 03/006558 discloses multilayer pigments based on glass flakes having a thickness of less than 1 $\mu$m.

JP 2002138019 discloses an eye-makeup cosmetic containing (a) a pearly-luster pigment comprising a glass flake having an average thickness of 0.1 to 3.0 $\mu$m, an average particle size of 1 to 700 $\mu$m and an aspect ratio of 5 to 500, whose surface is coated with a metal oxide, and (b) a film-forming emulsion polymer.

### Disclosure of the Invention

### Problems to be Solved by the Invention

The bright black color pigments described in the above-mentioned Patent Document Nos. 1 to 5 are not satisfactory in their brightness. Further, there is a problem in the pigments that the feeling and smooth spreading properties are inferior when used in the case of cosmetics containing these pigments.

With respect to the metallic pigment comprising glass flakes coated with a nickel alloy with a thickness of 0.04 $\mu$m or less and showing black appearance as described in Patent Document No.6, there is a problem that the hiding power is low and the coloration is weak. In this connection, if the thickness of the coating is thick, the metallic gloss becomes intense and the actually appearing color becomes glossy white gray to gray, which is not said to be glossy black color tone.

An object of the present invention is to provide a flaky black bright pigment having a high hiding power which is excellent in the feeling, smoothness and spread at the time of use of cosmetics containing the pigment.

### Means for Solving the Problems

A flaky black bright pigment of the present invention comprises a flaky base material having an average thickness of 1.3 to 5.0 $\mu$m, an average particle diameter of 1 to 800 $\mu$m and an aspect ratio of 3 to 500, wherein the flaky base material is a flaky C-glass base material or a flaky C-glass base material bearing an oxidized metal layer, and the surface of the base material is coated with a thin film having a thickness of 10 nm to 1.0 $\mu$m of triiron tetroxide and/or a low order titanium oxide.

The flaky glass can be produced by conventionally known methods, for example methods described in Japanese Examined Patent Publication No. 41-17148 and Japanese Examined Patent Publication No. 45-3541. That is, the flaky glass is produced by a method comprising extruding melted glass through a circular slit, injecting air into the inside of the glass, expanding the glass into a hollow cylinder form to make a thin and uniform glass film, and crushing the glass film. Also, examples of the known methods may include a method of forming flakes by blowing melted glass to a rotating disk and thereby quenching and shaping the glass into a flaky form as described in US Patent No. 5, 017, 207, and a method of forming flakes by blowing melted glass through a rotating slit into fibrous shape, hitting the resulting glass to a wall face, and thereby quenching and shaping the glass into a flaky form as described in US Patent No. 989,671. The flaky glass can be produced with a reduced cost since it is produced by melting economical starting materials. Also, in order to solidify the melted glass having free surface upon cooling, the surface is remarkably smooth and amorphous, and free from cleavage and therefore, there is no difference in level on the surface. Accordingly, the surface can show efficient bright feeling with a high reflectivity to visible light. Being different from flaky glass, mica has a cleavage property and difference in level on the surface, diffused reflection of light is caused, and in the case of observation from a certain direction, the brightness cannot be sufficiently exhibited. Also, because of the cleavage property, the feeling and smooth spreading properties are inferior in the case of application of cosmetics containing mica. The flaky glass of the mother material of the flaky black bright pigment is those having an average thickness of 1.3 to 5.0 $\mu$m, an average particle diameter of 1 to 800 $\mu$m, and an aspect ratio of 3 to 500; preferably those having an average particle diameter of 3 to 600 $\mu$m, and an average aspect ratio of 4 to 200; and furthermore preferably those having an average particle diameter of 5 to 500 $\mu$m, and an average aspect ratio of 5 to 100. The average particle diameter of the flaky black bright pigment can be measured by a laser diffracting scattering type of particle size distribution measurement apparatus, for example, Microtrac II (manufactured by Nikkiso Co. , Ltd.); the average thickness can be measured by calculating a simple average of measurement results of 50 pieces by a Shear type light interference microscope (Interphako, manufactured by Jena-Carl Zeiss); and the average aspect ratio can be measured by dividing the value of the above-mentioned average particle diameter by the value of the above-mentioned average thickness.

The flaky glass bearing a metal oxide layer is not particularly limited if a metal oxide layer is formed partially or entirely on the above-mentioned flaky glass. The metal oxide layer is a layer comprising a metal oxide, and examples of such a metal oxide may include one or more metal oxides selected from, for instance, silica, zirconium oxide, zinc oxide, and tin oxide.

The means for forming the metal oxide layer on the flaky glass is not particularly limited if it does not inhibit the purpose of the present invention, and may be selected appropriately from conventionally known coating means. For example, in the case where the metal oxide layer is a layer comprising silica, a metal oxide layer can be formed on the flaky glass by dispersing it in water of 65 to 75°C, adjusting the pH to 7 to 9 with a mineral acid or sodium hydroxide, dropwise adding sodium silicate and a mineral acid while keeping the liquid temperature and pH, and thereafter carrying out water washing, drying and further firing steps.

As the thin film material for coating on the surface of the flaky glass base material in the present invention is used triiron tetroxide (black iron oxide, magnetite $Fe_3O_4$) or a low order titanium oxide (black titanium oxide, $TiO_x$ wherein x is less than 2 and generally 1.5 to 1.8). A mixture of triiron tetroxide and a low order titanium oxide may be used and a laminate of a thin film of triiron tetroxide and a thin film of a low order titanium oxide may be used. In view of the safety and effect of dark interference color development, triiron tetroxide is preferably used. Adjustment of the thickness of the thin film in the coating makes it possible to give interference color. The thickness is 10 nm to 1.0 $\mu$m (1000 nm). If the thickness is thinner than 10 nm, black coloration is weak, and if it exceeds 1.0 $\mu$m, problems that it takes a long time to produce or that the coating layer is cracked may occur. If the thickness of the coating layer is 30 to 300 nm, beautiful interference color can be seen. If it exceeds 300 nm, the light absorption is intensified and the interference color becomes weak, however, the black coloration is intensified and the hiding power is increased.

It may be possible to use any method for coating the surface of the flaky glass base material with a thin film of triiron tetroxide or a low order titanium oxide. For example, such methods include a method of depositing a thin film of triiron tetroxide or a low order titanium oxide on the surface of a flaky glass base material by hydrolyzing salts, alkoxides, etc. of iron(II) or titanium(III) in wet manner in the presence of an oxidizing agent; a sputtering method; a CVD method; and a method of sticking a pigment to the flaky glass surface by using colloidal silica, silicon alkoxide, or a silane coupling agent as a binder. Further, there can be exemplified a method which is not a method of coating by using triiron tetroxide or a low order titanium oxide itself but involves reducing a formed thin film coating into triiron tetroxide or subtitanium oxide by a conventional manner. An example of this method is a method which involves forming a coating with diiron trioxide ($Fe_2O_3$) or titanium dioxide ($TiO_2$) and reducing it to triiron tetroxide ($Fe_3O_4$) or a low order titanium oxide ($TiO_x$ (x is less than 2)). More particularly, the surface of the flaky glass base material is previously coated with diiron trioxide and/or titanium dioxide, and converting into triiron tetroxide or a low order titanium oxide by firing in reducing atmosphere. This reduction reaction makes the thin film black and the black color type tone can be changed in accordance with the reducing conditions at that time. The surface of the flaky glass base material may be coated with a monolayer or two or more layers. In the case where two or more layers are formed in the coating, deeper black color and black color with the hiding property can be obtained, and the coloration of the interference color becomes more vivid, or effect of the changed interference color can be observed depending on an angle.

The above description explains the case that the flaky glass base material is used as the flaky base material, and a black bright pigment can be also produced in the same manner as in the case that the flaky base material is a flaky glass bearing a metal oxide layer.

Since the black bright pigment produced in such a manner has a flaky shape having smooth surface, the pigment regularly reflects light and is a dark to black type pigment giving the bright feeling. Particularly, with respect to the bright feeling, it is preferable that the brightness (L15 value) of the black bright pigment is 40 or higher.

A cosmetic exhibiting a black color with the bright feeling which has not been obtained so far and good touch feeling can be produced by adding the flaky black bright pigment to the cosmetic. The content of the flaky black bright pigment in the cosmetic is suitably 1 to 100% by mass. If it is less than 1% by mass, coloration of black color is weak.

In the case of a powder type cosmetic such as eye shadow, face color, etc. used as a loose powder, since it is mixed with human fat existing on the skin at the time of use, it may be 100%.

The above-mentioned flaky black bright pigment may be appropriately subjected to hydrophobic treatment in accordance with the purpose of the cosmetic. As the method for hydrophobic treatment, there can be exemplified a treatment with a silicone compound such as a methylhydrodiene polysiloxane, a highly viscid silicone oil and a silicone resin; a treatment with a surfactant such as an anionic surfactant and a cationic surfactant; a treatment with a polymer compound such as nylon, poly(methyl methacrylate), polyethylene, a fluoro resin, and a polyamino acid; a treatment with a perfluoro group-containing compound, lecithin, collagen, a metal soap, an oleophilic wax, and a partial or complete ester of a polyhydric alcohol; or a treatment by combining these treatments. However, the treatment is not limited to these exemplified treatments and any method for the hydrophobic treatment of a powder can be applicable.

The cosmetic may appropriately incorporate other components to be used commonly for cosmetics beside the black bright pigment of the present invention. Other components include an inorganic powder, an organic powder, a pigment, a coloring agent, an oil component, an organic solvent, a resin, and a plasticizer. Examples of the inorganic powder are talc, kaolin, sericite, muscovite, phlogopite, lepidolite, biotite, lithia mica, vermiculite, magnesium carbonate, calcium carbonate, diatomaceous earth, magnesium silicate, calcium silicate, aluminum silicate, barium silicate, barium sulfate, strontium silicate, metal tungstate, silica, hydroxyapatite, zeolite, boron nitride, and a ceramic powder.

The organic powder includes a nylon powder, a polyethylene powder, a polystyrene powder, a benzoguanamine powder, a poly(tetrafluoroethylene) powder, a distyrene-benzene polymer powder, an epoxy powder, and an acrylic powder.

The pigment includes microcrystalline cellulose, and inorganic white pigments such as titanium dioxide and zinc oxide; inorganic red type pigments such as iron oxide (red iron oxide) and iron titanate; inorganic brown type pigments such as $\gamma$-iron oxide; inorganic yellow type pigments such as yellow iron oxide and yellow earth; inorganic black type pigments such as black iron oxide and carbon black; inorganic violet type pigments such as mango violet and cobalt violet; inorganic green type pigments such as chromium oxide, chromium hydroxide, and cobalt titanate; inorganic blue type pigments such as ultramarine and prussian blue; pearl pigments such as titanium oxide-coated mica, titanium oxide-coated bismuth oxychloride, bismuth oxychloride, titanium oxide-coated talc, scaly foil, and colored titanium oxide-coated mica; and metal powder pigments such as aluminum powder and copper powder.

The coloring agent includes organic pigments such as Red No. 201, Red No. 202, Red No. 204, Red No. 205, Red No. 220, Red No. 226, Red No. 228, Red No. 405, Orange No. 203, Orange No. 204, Yellow No. 205, Yellow No. 401, and Blue No. 404; organic pigments of zirconium, barium, or aluminum lakes of Red No. 3, Red No. 104, Red No. 106, Red No. 227, Red No. 230, Red No. 401, Red No. 505, Orange No. 205, Yellow No. 4, Yellow No. 5, Yellow No. 202, Yellow No. 203, Green No. 3, or Blue No.1; and natural coloring agents such as chlorophyll and $\beta$-carotene.

Further, the oil component includes various kinds of hydrocarbons, silicone oils, higher fatty acids, esters of fats and oils, higher alcohols, and waxes, such as squalane, liquid paraffin, vaseline, microcrystalline wax, ozokerite, ceresin, myristic acid, palmitic acid, stearic acid, oleic acid, isostearic acid, cetyl alcohol, hexadecyl alcohol, oleyl alcohol, cetyl 2-ethylhexanoate, 2-ethylhexyl palmitate, 2-octyldodecyl myristate, neopentyl glycol di-2-ethylhexanoate, glycerol tri-2-ethylhexanoate, 2-octyldodecyl oleate, isopropyl myristate, glycerol triisostearate, tri(coconut oil fatty acid) glyceride, olive oil, avocado oil, beeswax, myristyl myristate, mink oil and lanoline.

Further, other components to be added to cosmetics include an organic solvent such as acetone, toluene, butyl acetate and acetic acid esters; a resin such as alkyd resin and urea resin; a plasticizer such as camphor and acetyl tributyl citrate; an ultraviolet absorbent, an antioxidant, a preservative, a surfactant, a moisture agent, a fragrance, water, alcohol, and a thickener.

The cosmetic may take various forms such as a powder, a cake-like form, a pencil-like form, a stick, an ointment, a liquid, an emulsion, and a cream. They may include, for example, make-up cosmetics such as a foundation, a lipstick, an eye shadow, cheek rouge, an eyeliner, a nail enamel, and a mascara; hair cosmetics such as hair color; facial cosmetics such as a lotion, an emulsion, and a cream.

Further, the flaky black bright pigment may be used as a filler for coating materials and resins to be kneaded therewith as well as a filler for films and inks in the same manner as in a conventional filler. In the use of the flaky black bright pigment as a coating material, a coating material composition containing said pigment is applied to a coating base material by a conventional method and cured to form a coating film, and this coating film is excellent in the extension on the coating base material, does not become uneven, and shows black color with fine color tone. In the case where the pigment is used for a resin, a resin composition containing the flaky black bright pigment is molded according to a conventional process, thereby to obtain a resin molded product (e.g. a resin film or the like) and the obtained resin molded product is free from color shading and shows black color with fine color tone. Further, in the case of using the pigment for inks, the writing or printing does not become uneven and shows black color with fine color tone. The content of the flaky black bright pigment in the coating composition, the molding resin composition, and the ink composition is preferably 1 to 70% by mass. If it is less than 1% by mass, the black coloration is weak and if it exceeds 70% by mass, mixing becomes difficult. The content of the flaky black bright pigment is more preferably 3 to 50% by mass.

**Effect of the Invention**

The flaky black bright pigment comprises a smooth and cleavage-free flaky glass as a mother material coated with the black bright pigment and is capable of showing black color with very brightness, and gives good touch feeling and smoothness.

If the flaky black bright pigment is used for a filler for cosmetics, coating materials, and resin molded products, it can give black color with brightness. If the pigment is used for a filler of inks, it can give writing and printing showing black color with brightness.

**Best Mode of the Embodiments of the Invention**

Hereinafter, the present invention will be described in more detail with reference to Examples, however, it is not intended that the present invention be limited to the illustrated Examples.

Examples

The brightness was evaluated by the following means for flaky black bright pigments produced in respective Examples and Comparative Examples.

(Evaluation of hue and brightness)

A proper amount of each flaky black bright pigment was added to an acrylic resin coating material (Acryl Auto Clear Super, manufactured by NIPPON PAINT Co. , Ltd. , solid matter about 50% by mass) so as to be 10% by mass in the resin, and well mixed and stirred, and then applied to a hiding power measurement paper by an applicator with intervals of 9 mil (9/1000 inch) and dried. The coated sheet was subjected to measurement of brightness L15 value at 15° angle, hue a value and b value by a multi-angle spectrocolorimeter (X-rite MA 68II, Color Techno System Corp.). As the brightness L 15 value is higher, the brightness is higher and gives more brightness.

(Evaluation of hiding power)

Each coated sheet of the hiding power measurement paper produced as described above was subjected to measurement of L value (Lw) in the white background and L value (Lb) in the black background by a color and color difference meter CR 300 manufactured by MINOLTA, and the value calculated by dividing the Lw value by the Lb value was employed as the hiding index. As the difference between the L value in the white background and the L value in the black background is lower (that is, the hiding index is nearer to 1), the hiding power is evaluated to be higher.

$$\text{Hiding  index = Lw/Lb}$$

(Eye Determination of Brightness)

Using above-mentioned each coated sheet for the hue and brightness measurement, the coated sheet was observed to determine the brightness in the case of eye observation by human being. The eye observation was carried out by five persons and the brightness was marked according to the following standards shown in Table 1 and the average of the points of five persons was calculated to evaluate the brightness according to the standard shown in Table 2.

Table 1

| Point | Impression of brightness |
|-------|--------------------------|
| 5 | very intense brightness |
| 4 | intense brightness |
| 3 | slight brightness |
| 2 | little brightness |
| 1 | no brightness |

Table 2

| Evaluation | Average point of impression |
| --- | --- |
| * | average 4.5 to 5.0 |
| ◎ | average 3.5 to 4.4 |
| ○ | average 2.5 to 3.4 |
| Δ | average 1.5 to 2.4 |
| × | average less than 1.5 |

(Examples 1 to 7)

As Reference Examples 1 to 3, flaky glass having prescribed thickness, particle size, and aspect ratio shown in Table 3 was produced by melting E glass ($SiO_2$: 53% by mass, $Al_2O_3$: 15% by mass, CaO: 21% by mass, MgO: 2% by mass, $B_2O_3$: 8% by mass, $Na_2O + K_2O$: 0.3% by mass) at 1, 300°C, blowing the melted glass into a cylindrical shape to draw the melted glass into a thin film, and cooling and solidifying the film in a specified thickness shown in Table 3, and crushing and classifying the film. As Examples 4 to 7, flaky glass having prescribed thickness, particle size, and aspect ratio shown Table 3 was produced by melting C glass ($SiO_2$: 65% by mass, $Al_2O_3$: 4% by mass, CaO: 14% by mass, MgO: 3% by mass, $B_2O_3$: 5% by mass, $Na_2O$: 8% by mass, $K_2O$: 1% by mass) at 1, 200°C , blowing the melted glass into a cylindrical shape to draw the melted glass into a thin film, and cooling and solidifying the film in a specified thickness shown in Table 3, and crushing and classifying the film.

Each flaky glass thus obtained (100 g) was added to and dispersed in 1 L of purified water, and kept under heating at 75°C in a thermostat water tank. The resulting solution was adjusted to a pH of about 3.2 with a diluted hydrochloric acid. After that, 10% iron (III) chloride solution was added. In this case, the pH was kept about 3.2 by a diluted sodium hydroxide solution, thereby to give a flaky glass of which surface was coated with $Fe_2O_3$. The addition amount of the 10% iron(III) chloride solution was adjusted so as to make the coating thickness approximately even. That is, in the case where the thickness of the mother flaky glass was 0.7 μm, the total addition amount of the 10% iron(III) chloride solution was adjusted to 2005 g; in the case the thickness was 1.3 μm, the total addition amount of the 10% iron(III) chloride solution was adjusted to 1080 g; and in the case the thickness was 5 μm, the total addition amount of the 10% iron(III) chloride solution was adjusted to 281 g. After completion of the addition, the flaky glass suspension solution was filtered, and the recovered residue was dried, the residue was previously fired at 600°C for 2 hours to obtain $Fe_2O_3$-coated flaky glass.

The $Fe_2O_3$-coated flaky glass was put in a platinum dish and heated at 530°C for 2 hours in a tubular furnace while hydrogen 10%-nitrogen 90% gas was circulated to reduce $Fe_2O_3$ on the surface of the flaky glass to black $Fe_3O_4$. The thickness of $Fe_3O_4$ thin film was about 250 nm for all of Examples 1 to 7.

With respect to each flaky black bright pigment, its brightness L15 value in the coating film was measured according to the above-mentioned means. The results are shown in Table 3 together with the average particle diameter (μm), the average thickness (μm), the average aspect ratio, color, eye observation brightness, and hiding index of the flaky black bright pigment.

(Reference Example 8)

100 g of the flaky glass produced in Example 3 was dispersed in ion exchange water of 70°C, and adjusted to pH 8.0 with a mineral acid, and then sodium silicate 30 g was dropwise added over a period of 1 hour while the temperature and pH of the solution being kept as they were.

After that, the solid matter was recovered by filtration and washed with water, dried, and fired at 600°C. In such a manner, silica-coated flaky glass having the prescribed thickness, particle size, and aspect ratio shown in Table 3 was produced. The silica-coated flaky glass was coated with $Fe_2O_3$ in the same manner as Example 3 to produce silica-coated flaky glass coated with $Fe_2O_3$.

Table 3

| Example | 1* | 2* | 3* | 4 | 5 | 6 | 7 | 8* |
| --- | --- | --- | --- | --- | --- | --- | --- | --- |
| Type of glass | E | E | E | C | C | C | C | E (silica-coated) |
| average particle diameter (μm) | 10 | 20 | 25 | 40 | 120 | 30 | 90 | 25 |
| Average thickness (μm) | 0.7 | 0.7 | 1.3 | 1.3 | 1.3 | 5.0 | 5.0 | 1.3 |
| Average aspect ratio | 14 | 29 | 19 | 31 | 92 | 6 | 18 | 19 |

(continued)

| Example | 1* | 2* | 3* | 4 | 5 | 6 | 7 | 8* |
|---|---|---|---|---|---|---|---|---|
| Color | black | black | black | black | black | black | black | black |
| L15 value | 53 | 60 | 71 | 63 | 77 | 66 | 78 | 68 |
| Brightness | ○ | ○ | ○ | ○ | ◎ | ○ | ◎ | ○ |
| Hiding index | 1.01 | 1.00 | 1.03 | 1.03 | 1.02 | 1.04 | 1.06 | 1.04 |

\* Reference Example

(Comparative Examples 1 to 3)

As Comparative Examples 1 and 2, black iron oxide coating was formed in the same manner as Example 1 to produce black color mica which was subjected similarly to the evaluation, except that mica shown in Table 5 was used in place of the flaky glass. The results are shown in Table 5. Also, as Comparative Example 3, commercially available mica black (mica coated with black iron oxide and titanium oxide; manufactured by Merck & Co., Inc.) was also evaluated similarly and the results are shown together in Table 5.

(Comparative Example 4)

Flaky glass (Micro Glass Fine Flake RCXFFX-1040 (9507), manufactured by Nippon Sheet Glass Co., Ltd. , C glass composition, average thickness 1.3 $\mu$m, average particle diameter 38 $\mu$m) 1.2 kg was added to 4 L of ion exchange water and stirred by a stirrer to obtain a slurry. After an aqueous solution of 0.5% by mass of tin(II) chloride 0.3 L was added to the slurry at normal temperature, the mixture was stirred for 5 minutes. The flaky glass was recovered by vacuum filtration and washed with ion-exchange water. The flaky glass was suspended in 4 L of pure water to obtain a slurry, and an aqueous solution of 2% by mass of palladium(II) chloride 0.01 L was added to the slurry, followed by stirring for 5 minutes . The flaky glass was recovered by vacuum filtration and washed with pure water.
While being stirred, 20 L of an electroless plating solution having the following composition as shown in Table 4 was heated to 70°C. The above-mentioned pretreated flaky glass 1.2 kg was suspended in the plating solution and stirred for 30 minutes. The product was recovered by vacuum filtration and washed with pure water and vacuum-dried at 150°C. The obtained product was sieved with a stainless mesh with 100 $\mu$m aperture. The obtained pigment had black color and showed metallic gloss. The nickel phosphorus alloy layer on the surface of the flaky glass was 0.02 $\mu$m in thickness.
The results of evaluation made in the same manner as the above-mentioned Examples are shown in Table 5.

Table 4
(Electroless plating liquid composition)

| | |
|---|---|
| sodium hypophosphite | 25 g/L |
| sodium hydrogen citrate | 15 g/L |
| ammonium acetate | 10 g/L |
| ammonium sulfate | 5 g/L |
| nickel sulfate | 25 g/L |
| lead nitrate | 30 mg/L |

Table 5

| Comparative Example | 1 | 2 | 3 | 4 |
|---|---|---|---|---|
| Type of flakes | mica 1 | mica 2 | mica 3 | flaky glass |
| Average particle diameter ($\mu$m) | 7 | 19 | 25 | 38 |
| Coating layer | black iron oxide | black iron oxide | black iron oxide + titanium oxide | nickel alloy |
| Color L15 value | black 20 | black 33 | black 37 | black 56 |
| Brightness | × | × | Δ | ○ |

(continued)

| Comparative Example | 1 | 2 | 3 | 4 |
|---|---|---|---|---|
| Hiding index | 1.02 | 1.02 | 1.01 | 1.10 |

Note)
mica 1; Y-1500, manufactured by Yamaguchi Mica Co., Ltd.
mica 2; Y-2000, manufactured by Yamaguchi Mica Co., Ltd.
mica 3; Mica Black, manufactured by Merck & Co., Inc., bearing coating layer.

It can be understood that black bright pigments comprising mica whose surface was coated with black iron oxide or with black iron oxide and titanium oxide scarcely showed brightness, while the black bright pigment comprising the flaky glass whose surface was coated with black iron oxide showed brightness compared to these Comparative Examples. Although the material of Comparative Example 4 comprising flaky glass whose surface was coated with nickel showed brightness, the hiding index exceeded 1.1, indicating an inferior hiding property.

(Examples 9 to 14)

Flaky glass (100 g) having a particle diameter of 90 $\mu$m and a thickness of 5.0 $\mu$m 100 g was added to and dispersed in 1 L of pure water, and kept under heating at 75˚C in a thermostat water tank. The solution was adjusted to about pH 3.2 with a mineral acid. After that, a prescribed amount of a 10% iron (III) chloride solution shown in Table 6 was added thereto. In this case, the pH was kept about 3.2 with a diluted sodium hydroxide solution so as to coat the flaky glass with $Fe_2O_3$. After completion of the addition, the flaky glass suspension was filtered and the recovered flaky glass was dried and fired at 600˚C for 2 hours to obtain $Fe_2O_3$-coated flaky glass.

The $Fe_2O_3$-coated flaky glass was put in a platinum dish and heated at prescribed reducing temperature as shown in Table 6 for 2 hours in a tubular furnace while hydrogen 10%-nitrogen 90% gas was circulated to reduce $Fe_2O_3$ to black $Fe_3O_4$. The thickness of $Fe_3O_4$ thin film was about 450 nm for all of Examples 9 to 11 and about 700 nm for Examples 12 to 14.

With respect to each flaky black bright pigment, its brightness L15 value in the coating film was measured according to the above-mentioned means. The results are shown in Table 6 together with the total addition amount of 10% iron chloride, reducing temperature, and brightness.

Table 6

| Example | 9 | 10 | 11 | 12 | 13 | 14 |
|---|---|---|---|---|---|---|
| Addition of 10% iron chloride (g) | 500 | 500 | amount 500 | 800 | 800 | 800 |
| Reduction (˚C) temperature | 400 | 500 | 530 | 400 | 500 | 530 |
| Color | dark gold | gold black | black | dark red | blackish red | black |
| L15 value | 72 | 68 | 60 | 68 | 66 | 57 |
| Brightness | * | ◎ | ○ | ◎ | ◎ | ○ |

According to comparison of Examples 9 and 12 or Examples 10 and 13, it is understood from Table 6 that the color can differ depending on the coating amount (addition amount of iron chloride). This is attributed to the interference color caused by the iron oxide coating is exhibited.

Also, based on comparison of Examples 9, 10 and 1 or Examples 12, 13, and 14, it is revealed that the color becomes more blackish as the reduction temperature is elevated to 400, 500, and 530˚C. It is supposedly attributed to the fact that the reduction of the iron oxide is further promoted and absorption of black is intensified to make the interference color invisible.

Thus, there are obtained dark color pigments with high L15 value and brightness and giving brightness.

(Example 15)

Flaky glass used in Example 4 and having a particle diameter of 40 $\mu$m and a thickness of 1.3 $\mu$m was suspended in a titanyl sulfate solution, and the suspension solution was heated and boiled for 1 hour to coat the surface of the flaky glass with titania and after filtration, washing water, and drying, the recovered flaky glass was heated at 600˚C for 30 minutes to obtain flaky glass coated with a titania coating. The titania-coated flaky glass was put in a platinum dish and heated at a temperature of 600˚C for 10 hours in a tubular furnace while ammonia gas was circulated to reduce the

coating $TiO_2$ to a low order black titanium oxide ($TiO_x$ wherein X is 1.7) to obtain a low order titanium oxide-coated flaky glass.

The L15 value of this low order titanium oxide-coated flaky glass was 67 and the brightness evaluated by panelists was ◎. The thickness of the low order titanium oxide coating was 150 nm.

Next, each flaky black bright pigment produced in the above-mentioned Examples and Comparative Examples was used for producing cosmetics for trial, and sensory tests for the use feeling of them were carried out. The items of the sensory tests were two; touch feeling at the time of application to the skin and the eyelashes and beautifulness of black (brightness), and the respective items were evaluated on the basis of 5 grades from 1 to 5. The evaluation criteria of the above items are shown in Tables 7 and 8.

Table 7

| Evaluation | Brightness |
|---|---|
| 1 | very intense brightness |
| 2 | intense brightness |
| 3 | brightness |
| 4 | little brightness |
| 5 | no brightness |

Table 8

| Evaluation | Touch feeling |
|---|---|
| 1 | smooth |
| 2 | rather smooth |
| 3 | ordinal |
| 4 | slightly rough touch feeling |
| 5 | rough touch feeling |

Ten panelists were employed in the following sensory tests for cosmetics. The brightness and touch feeling were evaluated on the basis of the average value of the evaluation points of these ten panelists. Additionally, to make the evaluation results easy to be understood, the following symbols shown in Table 9 are marked for the evaluation results.

Table 9

| | |
|---|---|
| ◎ : | 4.5 or higher, but up to 5.0 |
| ○ : | 3.5 or higher, but less than 4.5 |
| ● : | 2.5 or higher, but less than 3.5 |
| Δ : | 1.5 or higher, but less than 2.5 |
| × : | 1.0 or higher, but less than 1.5 |

(Example 16: Emulsion type mascara)

Emulsion type mascara was produced using the following respective components shown in the following Table 10.

Table 10

| | | | |
|---|---|---|---|
| (1) | hydroxyethyl cellulose | | 1.0 |
| (2) | methyl p-oxybenzoate | | 0.2 |
| (3) | glycerin | | 0.3 |
| (4) | polyethylene glycol with high polymerization degree (average molecular weight 2,000,000) | | 0.5 |
| (5) | pure water | | 65.0 |
| (6) | flaky black bright pigment of Example 4 | | 3.0 |
| (7) | triethanolamine | | 3.0 |
| (8) | stearic acid | | 5.0 |

(continued)

| (9) | beeswax | 9.0 |
| (10) | carnauba wax | 3.0 |
| (11) | paraffin wax | 10.0 |
| | | (% by mass) |

The components (1) to (5) were mixed and evenly dissolved by heating at 75°C. The black bright pigment of the component (6) was added to the mixture and evenly dispersed through a colloid mill. Further, the component (7) was mixed, dissolved, and heated at 75°C and further, the components (8) to (11) which are heated and evenly melted were added and the obtained mixture was emulsified and cooled to obtain an emulsion type mascara.

(Comparative Example 5)

An emulsion type mascara was produced in the same manner as Example 14, except that the iron oxide pigment of Comparative Example 1 was used in place of the black bright pigment (black iron oxide-containing flakes) of the component (6) in Example 16.

The results of the sensory tests for Example 16 and Comparative Example 5 are shown in Table 11.

Table 11

| | Brightness | Touch feeling |
| --- | --- | --- |
| Example 16 | ◎ | ◎ |
| Comparative Example 5 | Δ | Δ |

It is understood from Table 11 that the emulsion type mascara of the present invention is excellent in the brightness and touch feeling.

(Example 17: Eye shadow)

An eye shadow was produced from the following components shown in Table 12

Table 12

| (1) | talc | 21 |
| (2) | muscovite | 20 |
| (3) | flaky black bright pigment of Example 5 | 40 |
| (4) | pigment | 12 |
| (5) | squalane | 4 |
| (6) | cetyl 2-ethylhexanoate | 1.9 |
| (7) | sorbitan sesquioleate | 0.8 |
| (8) | preservative | 0.1 |
| (9) | fragrance | 0.2 (% by mass) |

The above-mentioned components (1) to (4) were mixed by a Henschel mixer and the components (5) to (9) which were heated and mixed were blown for mixing and then milled. The resulting mixture was discharged to a given medium-sized dish to obtain an eye shadow.

(Comparative Example 6)

An eye shadow was produced in the same manner as Example 17, except that the black iron oxide-coated mica of Comparative Example 2 was used in place of the flaky black bright pigment of Example 5 of the component (3).

The results of the sensory test of Example 17 and Comparative Example 6 are collectively shown in Table 13.

### Table 13

| | Brightness | Touch feeling |
|---|---|---|
| Example 17 | ◎ | ◎ |
| Comparative Example 6 | Δ | Δ |

It is understood from Table 13 that the eye shadow of the present invention is excellent in the brightness and touch feeling.

(Example 18: Eyeliner)

An eyeliner was produced from the following respective components shown in Table 14.

### Table 14

| | | |
|---|---|---|
| (1) | non-aqueous polymer dispersion | 25.0 |
| (2) | paraffin wax | 2.0 |
| (3) | bentonite | 3.0 |
| (4) | flaky black bright pigment of Example 5 | 2.0 |
| (5) | mica | 30.0 |
| (6) | Isopar | 38.0 |
| (8) | fragrance | q.s.(% by mass) |

The above-mentioned components (1) to (8) were heated at 85°C, stirred and mixed, and then cooled to room temperature and packed in an air-tight and brush-equipped container to produce an eyeliner.

(Comparative Example 7)

An eyeliner was produced in the same manner as Example 18, except that the black iron oxide-coated mica of Comparative Example 2 was used in place of the flaky black bright pigment of the component (4).

The results of the sensory test of Example 18 and Comparative Example 7 are collectively shown in Table 15.

### Table 15

| | Brightness | Touch feeling |
|---|---|---|
| Example 18 | ◎ | ◎ |
| Comparative Example 7 | Δ | Δ |

It is understood from Table 15 that the eyeliner of the present invention is excellent in the brightness and touch feeling.

(Reference Example 19: Nail color)

A nail color was produced from the following components shown in Table 16.

### Table 16

| | | |
|---|---|---|
| (1) | nitrocellulose | 18.0 |
| (2) | toluenesulfonamide resin | 6.0 |
| (3) | acetyl tributyl citrate | 6.0 |
| (4) | alkyl acrylate copolymer | 2.0 |
| (5) | isopropanol | 5.0 |
| (6) | benzyldimethylammonium hectorite | 2.0 |
| (7) | ethyl acetate | 20.0 |
| (8) | butyl acetate | q.s. |
| (9) | prussian blue | 0.1 |

(continued)

(10)    flaky black bright pigment of Example 2    10.0 (% by mass)

After the components (1) to (4) and components (9) and (10) were kneaded by a roller mill, the components (5) to (8) were added thereto, melted, diffused, and evenly dispersed, and then the resulting mixture was filled in a given container to obtain a nail color.

(Comparative Example 8)

A nail color was produced in the same manner as Example 19, except that the flaky black bright pigment of the component (10) was used in place of the black iron oxide-coated mica of Comparative Example 1.
The results of the sensory test of Example 19 and Comparative Example 8 are collectively shown in Table 17.

Table 17

|  | Brightness | Touch feeling |
|---|---|---|
| Example 19 | ○ | ◎ |
| Comparative Example 8 | Δ | Δ |

It is understood from Table 17 that the nail color of the present invention is excellent in the brightness and touch feeling.

(Reference Example 20: Oil-based stick foundation)

An oil-based stick foundation was produced from the following components shown in Table 18.

Table 18

| (1) | flaky black bright pigment of Example 1 | 13.0 |
|---|---|---|
| (2) | titania | 7.0 |
| (3) | kaolin | 20.0 |
| (4) | talc | 2.0 |
| (5) | mica | 26.0 |
| (6) | red iron oxide | 1.0 |
| (7) | yellow iron oxide | 3.0 |
| (8) | flaky black bright pigment of Example 1 | 0.5 |
| (9) | solid paraffin | 3.0 |
| (10) | microcrystalline wax | 7.0 |
| (11) | vaseline | 15.0 |
| (12) | dimethylpolysiloxane | 3.0 |
| (13) | squalane | 5.0 |
| (14) | isopropyl palmitate | 17.0 |
| (15) | antioxidant | q.s. |
| (16) | fragrance | q.s. (% by mass) |

The above-mentioned components (9) to (15) were dissolved at 85°C and the components (1) to (8) were added thereto. The mixture was mixed by a disper and dispersed by a colloid mill. After that, the component (16) was added thereto, and the mixture was degassed and poured into a container at 70°C, and cooled to obtain an oil-based stick foundation.
The oil-based stick foundation was excellent in the brightness and touch feeling.

(Example 21: Cheek rouge)

A cheek rouge was produced from the following respective components shown in Table 19.

Table 19

| (1) | kaolin | 24.0 |
|---|---|---|
| (2) | flaky black bright pigment of Example 4 | 0.1 |
| (3) | red iron oxide | 0.3 |
| (4) | Red 202 | 0.5 |
| (5) | ceresin | 15.0 |
| (6) | vaseline | 20.0 |
| (7) | liquid paraffin | 25.0 |
| (8) | isopropyl myristate | 15.0 |
| (9) | antioxidant | q.s.(% by mass) |

The components (1) to (4) were added to a portion of the component (7) and treated by a roller to prepare a pigment material. The remaining portion of the component (7) and the components (5), (6), (8), and (9) were heated and dissolved at 90˚C and mixed with the above pigment material and evenly dispersed by a homo-mixer and after being dispersed, the resulting mixture was filled in a given container to obtain an aimed cheek rouge.

The cheek rouge was excellent in brightness and touch feeling.

(Example 22: Lipstick)

A lipstick was produced from the following respective components shown in Table 20.

Table 20

| (1) | hydrocarbon wax | 20 |
|---|---|---|
| (2) | candelilla wax | 3 |
| (3) | glyceryl isostearate | 40 |
| (4) | liquid paraffin | 26.8 |
| (5) | titanium dioxide | 4 |
| (6) | flaky black bright pigment of Example 5 | 0.2 |
| (7) | organic pigment | 5.8 |
| (8) | fragrance | 0.2 (% by mass) |

The above-mentioned components (1) to (4) were dissolved at 85˚C and the components (5) to (7) were added thereto. The mixture was stirred and mixed, and after that, the component (8) was further added thereto, and the mixture was stirred, and packed in a prescribed container to obtain a lipstick.

The lipstick was excellent in the brightness and touch feeling.

Coating material compositions were produced using the above-mentioned flaky substances of Examples and Comparative Examples.

(Example 23: Black coating material composition)

A coating material component was produced from the following components.

At first, the components shown in the following Table 21 was dispersed for 60 minutes using a paint shaker to obtain a dispersion vehicle.

Table 21

| (1) | alkyd resin type varnish | 20.6 |
|---|---|---|
| (2) | melamine resin type varnish | 10.6 |
| (3) | Swazol | 15.6 |
| (4) | flaky black bright pigment of Example 4 | 15.6 (part by mass) |

The components shown in the following Table 22 were further added to and mixed with the above dispersion vehicle to produce a black color coating material composition.

Table 22

| | | | |
|---|---|---|---|
| (5) | alkyd resin type varnish | 26.3 | |
| (6) | melamine resin type varnish | 11.3 (part by mass) | |

(Comparative Example 9)

A black color coating material composition was produced in the same manner as Example 23, except that the black iron oxide-coated mica of Comparative Example 1 was used in place of the flaky black bright pigment of the component (4) in the coating material composition of Example 23.

The brightness was judged for the black color coating material compositions of Example 23 and Comparative Example 9. The results are shown in Table 23.

Table 23

| | Brightness |
|---|---|
| Example 23 | ◎ |
| Comparative Example 9 | × |

It was found from Table 23 that the black color coating material composition of the present invention has excellent brightness and very beautiful black color.

(Example 24: Molding resin composition and resin molded product)

98% by mass of methyl methacrylate copolymer beads and 2% by mass of the flaky black bright pigment of Example 7 were mixed and stirred by a Henschel mixer to obtain a molding resin composition. An acrylic resin molded product of 0.5 mm in thickness was produced from the composition by an extruder. The resin molded product showed brightness and clear black color.

(Comparative Example 10)

An acrylic resin molded product of 0.5 mm in thickness was produced in the same manner as Example 24, except that the black iron oxide-coated mica of Comparative Example 1 was used in place of the flaky black bright pigment used in Example 24. The resin molded product showed no brightness and mat black color.

As being understood from Example 24 and Comparative Example 10, the resin molded body of the present invention showed brightness and transparent and clear black color.

(Example 25: Ink composition)

The following respective components were sufficiently mixed to produce a black ink.

Table 24

| | | |
|---|---|---|
| (1) | flaky black bright pigment of Example 2 | 12 |
| (2) | ketone resin | 19 |
| (3) | ethanol | 59 |
| (4) | propylene glycol monomethyl ether | 10 (% by mass) |

When the ink composition was used for writing on white paper, the writing showed very beautiful brightness and black color.

**Industrial Applicability**

The black bright pigment of the present invention can be used as a filler for cosmetics, coating materials, resin molded products, and inks.

**Claims**

1. A black bright pigment comprising a flaky base material having an average thickness of 1.3 to 5.0 $\mu$m, an average particle diameter of 1 to 800 $\mu$m and an aspect ratio of 3 to 500, wherein the flaky base material is a flaky C-glass base material or a flaky C-glass base material bearing an oxidized metal layer, and the surface of the base material is coated with a film having a thickness of 10 nm to 1.0 $\mu$m of triiron tetroxide and/or a low order titanium oxide.

2. The black bright pigment according to claim 1 having a brightness L15 value of 40 or more.

3. The black bright pigment according to claim 1 or 2, wherein the film of triiron tetroxide and/or a low order titanium oxide is formed by previously coating the surface of the flaky base material with diiron trioxide and/or titanium dioxide and then converting into triiron tetroxide and/or a low order titanium oxide by firing in reducing atmosphere.

4. The black bright pigment according to claim 1, wherein the oxidized metal layer is a metal oxide layer comprising one or more metal oxide(s) selected from silica, zirconium oxide, zinc oxide, and tin oxide.

5. A cosmetic containing the black bright pigment according to any one of claims 1 to 4.

6. A coating material composition containing the black bright pigment according to any one of claims 1 to 4.

7. A coating film formed by applying the coating material composition according to claim 6 and curing the composition.

8. A molding resin composition containing the black bright pigment according to any one of claims 1 to 4.

9. A resin molded product obtained by molding the molding resin composition according to claim 8.

10. An ink composition containing the black bright pigment according to any one of claims 1 to 4.

**Patentansprüche**

1. Schwarzes glänzendes Pigment, umfassend ein flockiges Grundmaterial mit einer durchschnittlichen Dicke von 1,3 bis 5,0 $\mu$m, einem durchschnittlichen Teilchendurchmesser von 1 bis 800 $\mu$m und einem Seitenverhältnis von 3 bis 500, wobei das flockige Grundmaterial ein flockiges C-Glasgrundmaterial oder ein flockiges C-Glasgrundmaterial, das eine oxidierte Metallschicht trägt, ist und die Oberfläche des Grundmaterials mit einem Film mit einer Dicke von 10 nm bis 1,0 $\mu$m aus Trieisentetraoxid und/oder einem niederwertigen Titanoxid beschichtet ist.

2. Schwarzes glänzendes Pigment nach Anspruch 1 mit einem Glanz-L15-Wert von 40 oder mehr.

3. Schwarzes glänzendes Pigment nach Anspruch 1 oder 2, wobei der Film aus Trieisentetraoxid und/oder einem niederwertigen Titanoxid gebildet wird, indem die Oberfläche des flockigen Grundmaterials vorher mit Dieisentrioxid und/oder Titandioxid beschichtet und dann in Trieisentetraoxid und/oder ein niederwertiges Titanoxid durch Brennen in reduzierender Atmosphäre umgewandelt wird.

4. Schwarzes glänzendes Pigment nach Anspruch 1, wobei die oxidierte Metallschicht eine Metalloxidschicht, umfassend ein oder mehrere Metalloxid(e), ausgewählt aus Siliciumdioxid, Zirconiumdioxid, Zinkoxid und Zinnoxid, ist.

5. Kosmetisches Präparat, enthaltend das schwarze glänzendes Pigment nach einem der Ansprüche 1 bis 4.

6. Beschichtungsmaterialzusammensetzung, enthaltend das schwarze glänzendes Pigment nach einem der Ansprüche 1 bis 4.

7. Beschichtungsfilm, gebildet durch Aufbringen der Beschichtungsmaterialzusammensetzung nach Anspruch 6 und Härten der Zusammensetzung.

8. Formharzzusammensetzung, enthaltend das schwarze glänzendes Pigment nach einem der Ansprüche 1 bis 4.

9. Harzformprodukt, erhalten durch Formen der Formharzzusammensetzung nach Anspruch 8.

**10.** Tintenzusammensetzung, enthaltend das schwarze glänzende Pigment nach einem der Ansprüche 1 bis 4.


**Revendications**

1. Pigment noir brillant comprenant un matériau de base floconneux ayant une épaisseur moyenne de 1,3 à 5,0 μm, un diamètre particulaire moyen de 1 à 800 μm et un rapport d'aspect de 3 à 500, dans lequel le matériau de base floconneux est un matériau de base en verre de type C floconneux ou un matériau de base en verre de type C floconneux portant une couche métallique oxydée et la surface du matériau de base est revêtue d'un film ayant une épaisseur de 10 nm à 1,0 μm de tétroxyde de trifer et/ou d'un oxyde de titane de faible ordre.

2. Pigment noir brillant selon la revendication 1, ayant une valeur de brillance L15 de 40 ou plus.

3. Pigment noir brillant selon la revendication 1 ou 2, dans lequel le film de tétroxyde de trifer et/ou un oxyde de titane de faible ordre est formé en revêtant préalablement la surface du matériau de base floconneux avec du trioxyde de difer et/ou du dioxyde de titane et en la convertissant ensuite en tétroxyde de trifer et/ou en un oxyde de titane de faible ordre par calcination dans une atmosphère réductrice.

4. Pigment noir brillant selon la revendication 1, dans lequel la couche métallique oxydée est une couche d'oxyde métallique comprenant un ou plusieurs oxydes métalliques sélectionnés parmi la silice, l'oxyde de zirconium, l'oxyde de zinc et l'oxyde d'étain.

5. Produit cosmétique contenant le pigment noir brillant selon l'une quelconque des revendications 1 à 4.

6. Composition de matériau de revêtement contenant le pigment noir brillant selon l'une quelconque des revendications 1 à 4.

7. Film de revêtement formé par application de la composition de matériau de revêtement selon la revendication 6 et durcissement de la composition.

8. Composition de résine de moulage contenant le pigment noir brillant selon l'une quelconque des revendications 1 à 4.

9. Produit moulé dans une résine obtenu par moulage de la composition de résine de moulage selon la revendication 8.

10. Composition d'encre contenant le pigment noir brillant selon l'une quelconque des revendications 1 à 4.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 3913216 B
- JP 49128027 A
- JP 58164653 A
- JP 61019666 A
- JP 4145168 A
- JP 2002030232 A
- WO 03006558 A
- JP 2002138019 B
- JP 4117148 B
- JP 453541 A
- US 5017207 A
- US 989671 A